# DEMANDE DE BREVET EUROPEEN

(11) **EP 0 842 916 A1**
(43) Date de publication de la demande: **20.05.1998**
(21) Numéro de dépôt: 97402634.6
(22) Date de dépôt: 05.11.1997
(51) Int. Cl.: C07C 43/13, C07C 43/178, B01F 17/00, A61K 7/00, A61K 9/00, C07K 1/14

(54) **Composés bolaformes à têtes glycérol, procédés de préparation et utilisations**

(30) Priorité: 14.11.1996 FR 9613906
(71) Demandeur: CECA S.A., 92800 Puteaux (FR)
(72) Inventeur: Noiret, Nicolas, 35250 Saint Sulpice La Foret (FR); Patin, Henri, 35200 Rennes (FR); Rivaux, Yvan, 35700 Rennes (FR); Laffitte, Jean-Alex, 64000 Pau (FR); Brochette, Pascal, 64000 Pau (FR)
(74) Mandataire: Haicour, Philippe

(57) **Abrégé**

L'invention concerne des composés bolaformes symétriques constitués d'une chaîne hydrocarbonée en C₆-C₃₂ et deux têtes renfermant un motif ou un enchaînement de motifs glycéryles.

Elle a également pour objet deux procédés de préparation desdits composés l'un à partir d'un α,ω-alcanediol et l'autre à partir d'un alcool ω-acétylénique.

Les composés bolaformes peuvent être utilisés comme agent tensio-actif, d'encapsulation ou d'extraction des protéines.

## Description

La présente invention concerne des composés bolaformes à têtes glycérol, leurs procédés de préparation et leurs utilisations.

Les composés bolaformes ou bolaamphiphiles sont des molécules biamphiphiles constituées d'une chaîne carbonée hydrophobe de longueur relativement importante à chaque extrémité de laquelle est liée de manière covalente une tête hydrophile. Le terme bolaforme fait référence à l'arme de jet sud-américaine appelée "bolas" consistant en deux balles liées entre-elles par une corde ou une lanière de cuir.

Les composés bolaformes peuvent être symétriques ou dissymétriques selon que les têtes hydrophiles sont identiques ou différentes. En outre, la tête peut présenter un caractère anionique, cationique ou neutre (voir FUHRHOP J.H. et BACH R., Adv. in Supramol. Chem., vol. 2, pp. 25-63, 1992).

Les composés bolaformes présentent des propriétés tensio-actives. Ils sont notamment capables de former des agrégats micellaires, des liposomes et, à forte concentration, des phases cristallines liquides lamellaire ou hexagonale.

On a déjà proposé de préparer des composés bolaformes, en particulier symétriques à têtes neutres.

Dans FR-A-2 683 223, on propose des composés de formule RAEAR dans laquelle E représente une chaîne carbonée hydrophobe, A est un groupe de liaison et R est un résidu de sucre ou d'un dérivé de sucre.

KIM J.M. et THOMPSON D.H. (Langmuir, vol. 8, pp. 637-644, 1992) décrivent la préparation de composés bolaformes mono- ou bicaténaires possédant deux têtes formées d'un diéther de glycérol. La synthèse fait intervenir intermédiairement le composé bolaforme constitué d'une chaîne polyméthylène en C₁₆ ou C₂₀ portant à chaque extrémité un radical 1-glycéryle, ledit composé étant obtenu par bromation de l'α,ω-alcanediol correspondant, réaction avec le solkétal et hydrolyse.

Dans ce dernier procédé, le dérivé dibromé ne présente pas une réactivité élevée ce qui se traduit par un rendement faible, de l'ordre de 60 %. En outre, parce qu'on forme des produits secondaires indésirables, il est nécessaire de mettre en oeuvre une étape de purification.

YAMAUCHI K. et al. (Bull. Chem. Soc. Jpn., vol. 62, pp. 969-971, 1989) décrit la synthèse d'un agent anti-hypertenseur dans laquelle intervient, à titre de composé intermédiaire, le composé bolaforme constitué d'une chaîne polyméthylène en C₃₂ portant à chaque extrémité un radical 1-glycéryle.

La présente invention a pour objet de nouveaux composés bolaformes symétriques comprenant une chaîne carbonée hydrophobe, linéaire ou ramifiée, saturée ou insaturée ayant 6 à 32 atomes de carbone, et deux têtes renfermant un ou plusieurs motifs glycérol.

Un autre objet de l'invention concerne un procédé de préparation desdits composés bolaformes à partir de α,ω-alcanediol.

Un autre objet de l'invention concerne un procédé de préparation desdits composés bolaformes à partir d'un alcool ω-acétylénique.

Enfin, l'invention a pour objet l'utilisation des composés bolaformes précités en tant qu'agent tensio-actif, d'encapsulation ou d'extraction des protéines.

Les composés bolaformes selon l'invention ont pour formule :

R - O - A - O - R

dans laquelle :
R, identiques, représentent un motif ou un enchaînement de motifs de formule : 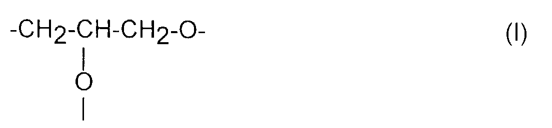 et/ou 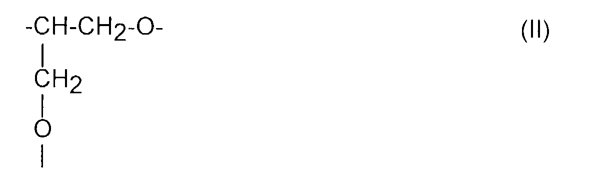 formules dans lesquelles la valence disponible de l'atome d'oxygène est liée à un atome d'hydrogène,
A représente une chaîne hydrocarbonée linéaire ou ramifié, saturée ou insaturée, renfermant 6 à 32 atomes de carbone, de préférence 14 à 24 atomes,
à l'exception des composés dans lesquels R représentent -CH₂-CH(OH)-CH₂OH et A est une chaîne hydrocarbonée linéaire saturée renfermant 16, 20 ou 32 atomes de carbone.

Dans la présente invention, l'expression "valence disponible" signifie toute valence qui n'est pas engagée dans une liaison avec un motif de formule (I) ou (II).

Les composés bolaformes de formule R-O-A-O-R précitée, dans laquelle R et A ont la signification donnée ci-avant, peuvent être préparés selon deux procédés.

Le premier procédé est caractérisé en ce qu'il comprend les étapes suivantes :
***a-*** réaction d'un α,ω-alcanediol, linéaire ou ramifié, saturé ou insaturé en C₆-C₃₂ et d'un composé de formule R'SO₂Cl dans laquelle R' représente un radical alkyle, linéaire ou ramifié, aryle, alkylaryle ou arylalkyle,
***b-*** réaction du produit de l'étape a- et d'un composé de formule R-OH dans laquelle R représente un motif ou un enchaînement de motifs de formule (I) et/ou (II) tels que définis précédemment, la valence disponible de l'atome d'oxygène étant liée à un groupement protecteur, et
***c-*** déprotection.

L'α,ω-alcanediol renferme de préférence 16 à 24 atomes de carbone. Il est avantageusement linéaire.

Le composé de formule R'SO₂Cl est de préférence choisi parmi les composés renfermant un radical alkyle tel que le chlorure de méthanesulfonyle, ou alkylaryle tel que le chlorure de p-toluènesulfonyle. On utilise avantageusement le chlorure de méthanesulfonyle.

L'étape a- est généralement mise en oeuvre en présence d'un solvant organique, par exemple chloré tel que le dichlorométhane, ou aromatique tel que le benzène, la pyridine ou la benzopyridine.

Lorsque le solvant ne contient pas d'atome d'azote, on ajoute avantageusement une amine telle que la triéthylamine ou la pyridine. De préférence, le solvant est le dichlorométhane, avantageusement anhydre, et l'amine est la triéthylamine.

On peut, en outre, utiliser un cosolvant tel que le 1,3-diméthyl-3,4,5,6-tétrahydro-2(1H)-pyrimidinone (DMPU).

Le rapport molaire α,ω-alcanediol/composé R'SO₂Cl est généralement compris entre 0,33 et 0,5 et de préférence 0,38 et 0,45.

Le composé R-OH contient généralement des groupes protecteurs choisis parmi les radicaux alkyles, arylalkyles tels que le benzyle, alkylidènes tels que l'isopropylidène ou acyles tels que l'acétyle.

De préférence, les composés R-OH qui contiennent un ou plusieurs motifs de formule (I) comportant deux atomes d'oxygène portant une valence disponible se présentent sous la forme de dérivés acétaliques. A titre d'exemple, on peut citer l'isopropylidèneglycérol (solkétal) et le diisopropylidènetriglycérol.

De préférence, les composés R-OH qui contiennent un ou plusieurs motifs de formule (II) comportant deux atomes d'oxygène portant une valence disponible se présentent sous la forme d'éthers. A titre d'exemple, on peut citer le glycérol-1,3-dibenzyléther.

L'étape b- est généralement mise en oeuvre en présence d'un solvant organique, par exemple choisi parmi les hydrocarbures tels que le n-pentane ou le n-hexane, les éthers tels que l'éther éthylique ou de pétrole et les solvants aromatiques tels que le toluène et d'une base telle que la potasse.

On peut avantageusement utiliser un catalyseur de transfert de phase tel que les ammonium quaternaires connus de l'homme du métier. A titre d'exemple, on peut citer les halogénures de tétrabutylammonium ou d'hexadécyltriméthylammonium.

L'eau générée au cours de cette étape peut éventuellement être éliminée par exemple par distillation azéotropique. Dans ce cas, le solvant précité est avantageusement le toluène.

Le rapport molaire du produit de l'étape a- au composé R-OH est généralement compris entre 0,25 et 0,5 et de préférence 0,33 à 0,5.

L'étape c- de déprotection peut être effectuée de deux manières selon la nature des groupes protecteurs.

Selon la première variante, les composés bolaformes renfermant des groupes acétaliques sont hydrolysés par catalyse acide hétérogène, par exemple en présence d'une résine à caractère acide et d'un solvant organique, et chauffage au reflux du solvant.

De préférence, on utilise la résine DOWEX 50X8 (ACROS ORGANICS) et l'acétonitrile.

Selon la deuxième variante, les composés bolaformes renfermant des groupes éthers ou esters sont déprotégés selon les méthodes connues de l'homme du métier. A titre d'exemple, on peut citer la déprotection des groupes éthers, notamment benzyliques, par hydrogénation catalytique et celle des groupes esters par saponification.

Le deuxième procédé de préparation des composés bolaformes précités est caractérisé en ce qu'il comprend les étapes suivantes :
***a-*** réaction d'un alcool ω-acétylénique en C₄-C₁₆ et d'un composé de formule R'SO₂Cl, R' ayant la signification donnée ci-avant,
***b-*** réaction du produit de l'étape a- et d'un composé de formule R-OH ayant la signification donnée ci-avant,
***c-*** couplage oxydant en présence d'un sel de cuivre,
***d-*** réduction, et, le cas échéant, déprotection.

L'alcool ω-acétylénique renferme de préférence 8 à 14 atomes de carbone. Il est généralement linéaire ou ramifié et, de préférence, linéaire. Il peut, en outre, renfermer une ou plusieurs insaturations.

L'alcool ω-acétylénique est généralement obtenu par bromation et déshydrobromation à partir d'un alcool ω-éthylénique dans les conditions connues de l'homme du métier qui ne constituent pas un objet de l'invention.

Le composé de formule R'SO₂Cl est tel que défini à l'étape a- du procédé décrit ci-avant. Il est généralement mis en oeuvre dans les mêmes conditions que ladite étape a-.

Le composé de formule R-OH est tel que défini à l'étape b- du procédé décrit ci-avant. Il est généralement mis en oeuvre dans les mêmes conditions que ladite étape b-.

Le sel de cuivre est de préférence choisi parmi les sels de cuivre I ou II de formule CuX ou CuX₂ dans laquelle X représente Cl, Br, I, CH₃COO ou CN. De préférence, on utilise le chlorure de cuivre ou l'acétate de cuivre.

Dans l'étape c- on peut avantageusement mettre en oeuvre un oxydant tel que l'oxygène.

L'étape c- est généralement mise en oeuvre en présence d'un solvant tel que la pyridine ou le 1,2-di(diméthylamino)éthane.

Le rapport molaire du produit de l'étape b- au sel de cuivre est généralement compris entre 0,33 et 10 et de préférence entre 0,66 et 1.

La réduction selon l'étape d- est généralement réalisée par hydrogénation catalytique des liaisons acétyléniques, par exemple au moyen d'un catalyseur au palladium supporté sur du charbon en présence d'un alcool en C₁-C₃.

Dans le cas des composés bolaformes renfermant des groupes éthers du type alkylaryle, notamment benzylique, l'étape de réduction permet également d'effectuer la déprotection.

Dans le cas des composés bolaformes renfermant des groupes éthers autres qu'alkylaryles ou esters, il est nécessaire de procéder, en outre, à une étape de déprotection, laquelle déprotection est généralement effectuée dans les conditions citées ci-avant.

Les composés bolaformes obtenus à l'issue des procédés qui viennent d'être décrits présentent des propriétés tensio-actives et cristal liquide amphotrope.

Ces composés possèdent également la faculté de former des vésicules sphériques ou cylindriques de taille relativement importante, de l'ordre de 5 µm de diamètre et jusqu'à 150 µm de longueur. La température, inférieure ou supérieure à la transition cristal liquide-cristal liquide L_{β'}>↔L_{α}, permet de moduler la rigidité et la perméabilité des vésicules formées. Une telle propriété peut être mise avantageusement à profit pour l'encapsulation et le relargage contrôlé de principes actifs, notamment dans le domaine de la pharmacie ou de la cosmétique, ainsi que pour l'extraction et l'encapsulation de protéines.

Les exemples qui suivent permettent d'illustrer l'invention sans toutefois la limiter.

Dans les exemples, on utilise les méthodes d'analyse ci-après :

### Résonance magnétique nucléaire (RMN)

Les spectres sont réalisés à 400,13 MHz (¹H) et 100,61 MHz (¹³C). Les déplacements chimiques sont exprimés en ppm et les constantes de couplage (J) en Hz.

L'aspect des signaux est noté : large (I), singulet (s), doublet (d), triplet (t), quadruplet (q), quintuplet (qt) et massif (ma).

### Spectrométrie de masse (SM)

Les spectres sont réalisés en ionisation chimique NH₃.

Les valeurs sont exprimées en unités de masse atomique m/z et les intensités en % du pic de base sont indiquées entre parenthèses.

### Chromatographie

Le R_{F} est mesuré par chromatographie sur couche mince de silice (60F₂₅₄ ; MERCK).

Les spots de migration sont révélés par pulvérisation d'une solution éthanolique d'acide phosphomolybdique (60 g/l).

### EXEMPLE 1

### a- Synthèse du 1,10-diméthanesulfonate de n-décanediyle

Dans un ballon bicol de 50 ml contenant 1 g (5,74 mmoles) de 1,10-décanediol dissous dans 6 ml de 1,3-diméthyl-3,4,5,6-tétrahydro-2(1H)-pyrimidinone, on introduit 4 ml de dichlorométhane et 1,72 g (17,21 mmoles) de triéthylamine en prenant soin de contrôler la solubilité du diol.

Le mélange réactionnel est refroidi (0°C à -10°C) et on ajoute goutte à goutte 1,44 g (12,63 mmoles) de chlorure de méthanesulfonyle.

Après 2 heures, on lave le mélange successivement avec une solution aqueuse d'HCl froid (de l'ordre de 0°C) à 10 % en poids, de Na₂CO₃ saturée et de NaCI saturée.

La phase organique est séchée, le solvant est distillé et le résidu est chromatographié sur une colonne de gel de silice (éluant: gradient éther de pétrole/éther). On obtient le 1,10-diméthanesulfonate de n-décanediyle avec un rendement de 54 % en mole calculé sur la base du diol de départ. Les caractéristiques de ce composé sont les suivantes : 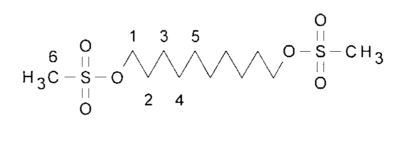
Solide blanc
M = 330,46 g.mol⁻¹ ; C₁₂H₂₆O₆S₂
R_{F} = 0,27 (éther de pétrole/éther; 8/2 v:v)
Point de fusion = 75°C (éther/dichlorométhane)
¹H RMN (CDCl₃) : 1,30-1,40 (ma, 12H, H₃ → H₅) ; 1,71-1,78 (qt, 4H, H₂, ^{J}H₂-H₁ = 6,6 ; ^{J}H₂-H₃ = 7,4) ; 3,01 (s, 6H, H₆) ; 4,22 (t, 4H, H₁, ^{J}H₁-H₂ = 6,6).
¹³C RMN (CDCl₃) : 25,37 (C₃) ; 28,93 ; 29,09 ; 29,23 (C₂, C₄, C₅) ; 37,36 (C₆) ; 70,18 (C₁).

### b- Synthèse du 1,1-O-(décane-1,10-diyl)-bis-(rac-isopropylidèneglycérol)

Dans un ballon bicol de 50 ml muni d'un réfrigérant, on introduit successivement 1,98 g (15 mmoles) de solketal, 12 ml d'une solution aqueuse de potasse à 33 % en poids et 0,24 g (0,75 mmole) de bromure de tétrabutylammonium.

Le mélange est agité à une température de l'ordre de 20 à 25°C.

On introduit goutte à goutte 2,47 g (7,5 mmoles) de 1,10-diméthanesulfonate de n-décanediyle. Le mélange est chauffé à 100°C sous une agitation de l'ordre de 1100 RPM pendant 15 heures.

Le mélange est décanté et la phase organique est chromatographiée sur une colonne de gel de silice (éluant: gradient éther de pétrole/éther). On obtient le 1,1-O-(décane-1,10-diyl)-bis-(rac-isopropylidèneglycérol) avec un rendement de 60 % en mole calculé sur la base du diméthylsulfonate de départ.

Ce composé présente les caractéristiques suivantes : 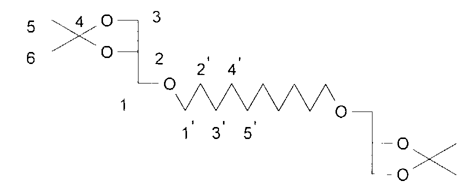
Huile incolore
M = 402,58 g.mol⁻¹ ; C₂₂H₄₂O₆
R_{F} = 0,86 (éther de pétrole/éther; 8/2 v:v)
¹H RMN (CDCl₃) : 1,25-1,34 (ma, 12H, H_{3'} → H_{5'}) ; 1,36 (s, 6H, H₅) ; 1,42 (s, 6H, H₆) ; 1,52-1,61 (ma, 4H, H_{2'}) ; 3,33-3,51 (ma, 8H, H₁ et H_{1'}) ; 3,73 (dd, 2H, H₃ₐ) ; 4,06 (dd, 2H, H_{3b}) ; 4,27 (qt, 2H, H₂).
¹³C RMN (CDCl₃) : 25,42 (C₅) ; 26,09 (C_{2'}) ; 26,77 (C₆) ; 29,41; 29,53 ; 29,62 ; 29,68 (C_{3'} → C_{5'}) 66,92 (C₃) ; 71,87 ; 71,95 (C₁ et C_{1'}) ; 74,81 (C₂) ; 109,28 (C₄).

### c- Synthèse du 1,1-O-(décane-1,10-diyl)-bis-(rac-glycérol)

Dans un ballon de 10 ml muni d'un réfrigérant, on introduit 280 mg (0,7 mmole) de 1,1-O-(décane-1,10-diyl)-bis-(rac-isopropylidèneglycérol), 26 mg (0,14 méq.) de résine acide (DOWEX 50X8 ; ACROS ORGANICS) et 4 ml d'acétonitrile.

Le mélange est chauffé au reflux pendant 4 heures sous agitation. L'acétonitrile est éliminé et le résidu est repris par 5 ml de méthanol et filtré.

Après élimination du méthanol sous pression réduite, on récupère 187 mg (rendement 83 %) de 1,1-O-(décane-1,10-diyl)-bis-(rac-glycérol) ayant les caractéristiques suivantes : 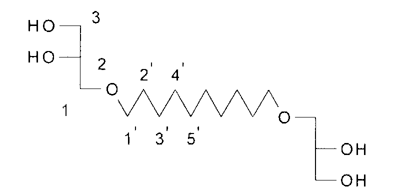
Solide blanc
M = 322,44 g.mol⁻¹ ; C₁₆H₃₄O₆
R_{F} = 0,70 (méthanol)
Point de fusion = 75°C (méthanol/dichlorométhane)
¹H RMN (CD₃OD) : 1,20-1,30 (ma, 12H, H_{3'} → H_{5'}) ; 1,46-1,53 (ma, 4H, H_{2'}) ; 3,31-3,39 (ma, 8H, H₁ et H_{1'}) ; 3,42 (dd, 2H, H₃ₐ, J_{H3a-H3b} = 11,2 ; J_{H3a}H2 = 6,1) ; 3,49 (dd, 2H, H_{3b}, J_{H3b-H3a} = 11,2 ; J_{H3b-H2} = 5,1); 3,66 (qt, 2H, H₂) ; 4,51 (I, 4H, OH).
¹³C RMN (CD₃OD) : 28,10 (C_{2'}) ; 31,48 ; 31,57 ; 31,61 (C_{3'} → C_{5'}) ; 65,51 (C₃) ; 73,15 (C₂) ; 73,51 (C₁) ; 74,13 (C_{1'}).
SM : 340,44 (100) [M + NH₄]⁺ ; 323,12 (57,3) [M + H]⁺

| | | | |
|---|---|---|---|
| Analyse élémentaire (C₁₆H₃₄O₆) | (% calculés) | C = 59,60 ; | H = 10,63 |
| | (% trouvés) | C = 59,37 ; | H = 10,77 |

### EXEMPLE 2

### a- Synthèse du 1,10-diméthanesulfonate de n-décanediyle

On procède dans les conditions de l'exemple 1 (étape a).

### b- Synthèse du 2,2-O-(décane-1,10-diyl)-bis-(1,3-dibenzylglycérol)

Dans un ballon tricol de 250 ml muni d'un dispositif Dean et Stark surmonté d'un réfrigérant, on introduit 3,26 g (12 mmoles) de glycérol-1,3-dibenzyléther, 84 ml de toluène et 5,9 g (106 mmoles) de potasse en poudre.

Le mélange réactionnel est chauffé au reflux du toluène pendant 2 heures pour éliminer l'eau par distillation azéotropique. On ajoute goutte à goutte 1,98 g (6 mmoles) de 1,10-diméthanesulfonate de n-décanediyle en solution dans 6 ml de toluène et on maintient au reflux pendant 4 heures.

Au mélange réactionnel obtenu, on ajoute 40 ml d'eau permutée et on extrait trois fois avec de l'éther éthylique. Les phases organiques sont rassemblées, séchées (MgSO₄) et l'huile jaune obtenue (4,78 g) est chromatographiée sur une colonne de gel de silice (éluant : gradient éther de pétrole/éther).

On isole ainsi 1,96 g (rendement : 48 %) de 2,2-O-(décane-1,10-diyl)-bis-(1,3-dibenzylglycérol) ayant les caractéristiques suivantes : 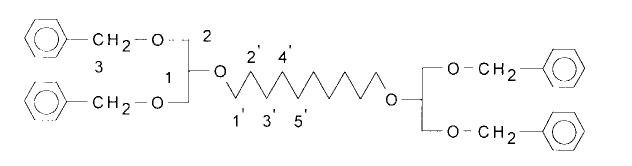
Huile incolore
M = 682,95 ; C₄₄H₅₈O₆
R_{F} = 0,67 (éther de pétrole/éther ; 1/1 v:v)
¹H RMN (CDCl₃) : 1,25-1,34 (ma, 12H, H_{3'} → H_{5'}) ; 1,55-1,63 (ma, 4H, H_{2'}) ; 3,44-3,64 (ma, 14H, H₁, H₂ et H_{1'}) ; 4,51 (s, 8H, H₃) ; 7,25-7,33 (ma, 20H, Hₐᵣₒₘ).
¹³C RMN (CDCl₃) : 26,09 (C_{2'}) ; 29,48 ; 29,76 (C_{3'} → C_{5'}) ; 70,10 (C₂) ; 70,58 (C_{1'}) ; 73,33 (C₃) ; 77,90 (C₁) ; 127,44 ; 127,5 ; 127,58 ; 128,3 ; 138,34 (C_{arom.}).

### c- Synthèse du 2,2-O-(décane-1,10-diyl)-bis-(glycérol)

Dans un ballon tricol de 50 ml, on introduit 1,65 g (2,42 mmoles) de 2,2-O-décyl-bis-(1,3-dibenzylglycérol), 0,12 g de catalyseur palladium/charbon (10 % en poids de palladium) et 15 ml de méthanol.

Le mélange est placé sous atmosphère d'hydrogène pendant 12 heures, sous agitation et à 20-25°C.

Après filtration et élimination du méthanol, on obtient 630 mg (rendement = 81 %) de 2,2-O-(décane-1,10-diyl)-bis-(glycérol) ayant les caractéristiques suivantes : 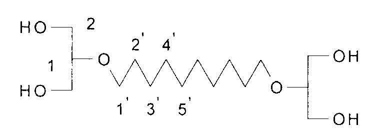
Solide blanc
M = 322,44 g.mol⁻¹ ; C₁₆H₃₄O₆
R_{F} = 0,69 (méthanol)
Point de fusion : 95°C (méthanol/dichlorométhane)
¹H RMN (CD₃OD) : 1,20-1,30 (ma, 12H, H_{3'} → H_{5'}) ; 1,45-1,51 (ma, 4H, H_{2'}) ; 3,44-3,54 (ma, 14H, H₁, H₂ et H_{1'}) ; 4,52 (I, 4H, OH).
¹³C RMN (CD₃OD) : 28,03 (C_{2'}) ; 31,51 ; 31,57 ; 32,02 (C_{3'}→C_{5'}) ; 63,32 (C₂) ; 72,32 (C_{1'}) ; 83,17 (C₁).
SM : 340,16 (100) [M + NH₄]⁺ ; 323,10 (73,8) [M + H]⁺

| | | | |
|---|---|---|---|
| Analyse élémentaire (C₁₆H₃₄O₆) | (% calculés) | C = 59,60 ; | H = 10,63 |
| | (% trouvés) | C = 59,54 ; | H = 10,79 |

### EXEMPLE 3

On procède dans les conditions de l'exemple 1 modifié en ce que l'on utilise le 1,12-dodécanediol.

A l'issue de l'étape a), on obtient le 1,12-diméthanesulfonate de n-dodécanediyle (rendement = 56 %) ayant les caractéristiques suivantes : 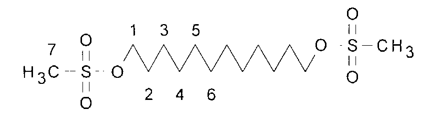
Solide blanc
M = 358,51 g.mol⁻¹ ; C₁₄H₃₀O₆S₂
R_{F} = 0,31 (éther de pétrole/éther; 2/8 v:v)
Point de fusion : 83°C (éther/dichlorométhane)
¹H RMN (CDCl₃) : 1,27-1,39 (ma, 16H, H₃ → H₆) ; 1,70-1,78 (q, 4H, H₂, J_{H2-H1} = 6,6) ; J_{H2-H3} = 7,4) ; 2,99 (s, 6H, H₇) ; 4,22 (t, 4H, H₁, J_{H1-H2} = 6,6).
¹³C RMN (CDCl₃) : 25,38 ; 25,73 (C₃) ; 28,98 ; 29,09 ; 29,35 ; 29,39 (C₂, C₄ → C₆) ; 37,33 (C₇) ; 70,25 (C₁).

A l'issue de l'étape b), on obtient le 1,1-O-(dodécane-1,12-diyl)-bis-(rac-isopropylidèneglycérol) (rendement = 58 %) ayant les caractéristiques suivantes : 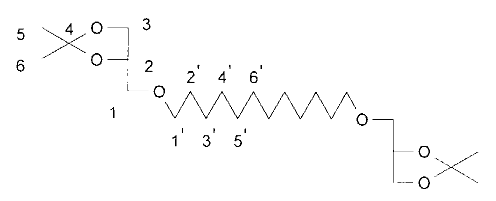
Huile incolore
M = 430,63 g.mol⁻¹ ; C₂₄H₄₆O₆
R_{F} = 0,88 (éther de pétrole/éther ; 2/8 v:v)
¹H RMN (CDCl₃) : 1,25-1,34 (ma, 16H, H_{3'} → H_{6'}) ; 1,35 (s, 6H, H₅) ; 1,41 (s, 6H, H₆) ; 1,50-1,60 (ma, 4H, H_{2'}) ; 3,33-3,50 (ma, 8H, H₁ et H_{1'}) ; 3,74 (dd, 2H, H₃ₐ) ; 4,06 (dd, 2H, H_{3b}) ; 4,24 (qt, 2H, H₂).
¹³C RMN (CDCl₃) : 25,45 (C₅) ; 26,10 (C_{2'}) ; 26,80 (C₆) ; 29,43 ; 29,52 ; 29,62 ; 29,70 (C_{3'} → C_{6'}) ; 66,89 (C₃) ; 71,85 ; 71,93 (C₁ et C_{1'}) ; 74,82 (C₂) ; 109,27 (C₄).

A l'issue de l'étape c), on obtient le 1,1-O-(dodécane-1,12-diyl)-bis-(rac-glycérol) (rendement = 86 %) ayant les caractéristiques suivantes : 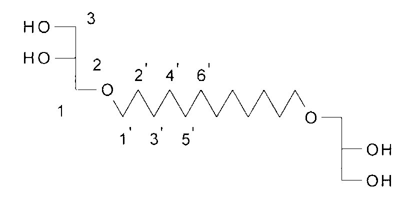
Solide blanc
M = 350,50 g.mol⁻¹ ; C₁₈H₃₈O₆
R_{F} = 0,72 (méthanol)
Point de fusion : 82°C (méthanol/dichlorométhane)
¹H RMN (CD₃OD) : 1,21-1,32 (ma, 16H, H_{3'} → H_{6'}) ; 1,45-1,53 (ma, 4H, H_{2'}) ; 3,30-3,39 (ma, 8H, H₁ et H_{1'}) ; 3,41 (dd, 2H, H₃ₐ) ; 3,49 (dd, 2H, H_{3b}) ; 3,65 (qt, 2H, H₂) ; 4,50 (I, 4H, OH).
¹³C RMN (CD₃OD) : 28,08 (C_{2'}) ; 31,48 ; 31,58 ; 31,60; 31,61 (C_{3'} → C_{6'}) ; 65,46 (C₃) ; 73,09 (C₂) ; 73,50 (C₁) ; 74,09 (C_{1'}).
SM : 368,50 (100) [M + NH₄]⁺ ; 350,97 (49,9) [M + H]⁺

| | | | |
|---|---|---|---|
| Analyse élémentaire (C₁₈H₃₈O₆) | (% calculés) | C = 61,68 ; | H = 10,93 |
| | (% trouvés) | C = 61,63 ; | H = 11,14 |

### EXEMPLE 4

On procède dans les conditions de l'exemple 2 modifié en ce que l'on utilise le 1,12-dodécane diol.

A l'issue de l'étape b) on obtient le 2,2-O-(dodécane-1,12-diyl)-bis-(1,3-dibenzylglycérol) (rendement = 51 %) ayant les caractéristiques suivantes : 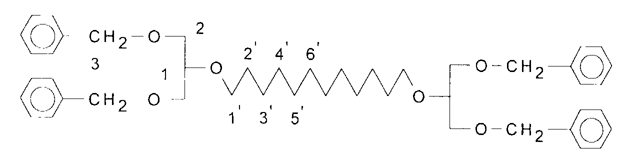
Huile incolore
M = 711,0 g.mol⁻¹ ; C₄₆H₆₂O₆
R_{F} = 0,70 (éther de pétrole/éther ; 1/1 v:v)
¹H RMN (CDCl₃) : 1,23-1,32 (ma, 16H, H_{3'} → H_{6'}) ; 1,54-1,62 (ma, 4H, H_{2'}) ; 3,43-3,62 (ma, 14H, H₁, H₂ et H_{1'}) ; 4,49 (s, 8H, H₃) ; 7,24-7,32 (ma, 20H, H_{arom.}).
¹³C RMN (CDCl₃) : 26,12 (C_{2'}) ; 29,51; 29,80 (C_{3'} → C_{6'}) ; 70,12 (C₂) ; 70,61 (C_{1'}) ; 73,35 (C₃) ; 77,93 (C₁) ; 127,46 ; 127,52 ; 127,59 ; 128,30 ; 138,36 (C_{arom.}).

A l'issue de l'étape c), on obtient le 2,2-O-(dodécane-1,12-diyl)-bis-(glycérol) (rendement = 87 %) ayant les caractéristiques suivantes : 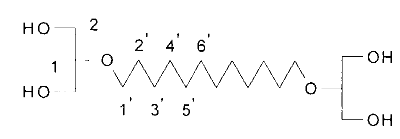
Solide blanc
M = 350,50 g.mol⁻¹ ; C₁₈H₃₈O₆
R_{F} = 0,72 (méthanol)
Point de fusion : 97°C (méthanol/dichlorométhane)
¹H RMN (CD₃OD) : 1,18-1,30 (ma, 16H, H_{3'} → H_{6'}) ; 1,45-1,52 (ma, 4H, H_{2'}) ; 3,44-3,55 (ma, 14H, H₁, H₂ et H_{1'}) ; 4,51 (I, 4H, OH).
¹³C RMN (CD₃OD) : 28,06 (C_{2'}) ; 31,53 ; 31,62 ; 32,06 (C_{3'} → C_{6'}) ; 63,39 (C₂) ; 72,29 (C_{1'}) ; 83,17 (C₁).
SM : 368,47 (100) [M + NH₄]⁺ ; 351,37 (59,0) [M + H]⁺

| | | | |
|---|---|---|---|
| Analyse élémentaire (C₁₈H₃₈O₆) | (% calculés) | C = 61,68 ; | H = 10,93 |
| | (% trouvés) | C = 61,98 ; | H = 11,19 |

### EXEMPLE 5

On procède dans les conditions de l'exemple 1 modifié en ce que l'on utilise le 1,16-hexadécanediol.

A l'issue de l'étape a), on obtient le 1,16-diméthanesulfonate de n-hexadécanediyle (rendement = 65 %) ayant les caractéristiques suivantes : 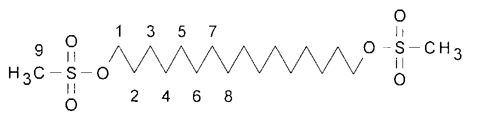
Solide blanc
M = 414,62 g.mol⁻¹ ; C₁₈H₃₈O₆S₂
R_{F} = 0,62 (éther)
Point de fusion : 93°C (éther/dichlorométhane)
¹H RMN (CDCl₃) : 1,23-1,33 (ma, 20H, H₄ → H₈) ; 1,36-1,41 (ma, 4H, H₃) ; 1,71-1,78 (ma, 4H, H₂) ; 3,01 (s, 6H, H₉) ; 4,22 (t, 4H, H₁, J_{H1-H2} = 6,6).
¹³C RMN (CDCl₃) : 25,45 (C₃) ; 29,06 ; 29,16 ; 29,45 ; 29,54 ; 29,62 ; 29,65 (C₂, C₄ → C₈) ; 37,38 (C₉) ; 70,30 (C₁).

A l'issue de l'étape b), on obtient le 1,1-O-(hexadécane-1,16-diyl)-bis-(rac-isopropylidèneglycérol) (rendement = 68 %) ayant les caractéristiques suivantes : 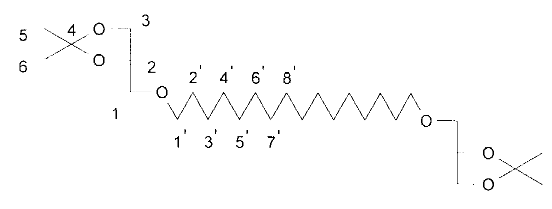
Solide blanc
M = 486,74 g.mol⁻¹ ; C₂₈H₅₄O₆
R_{F} = 0,84 (éther)
¹H RMN (CDCl₃) : 1,25-1,35 (ma, 24H, H_{3'} → H_{8'}) ; 1,36 (s, 6H, H₅) ; 1,42 (s, 6H, H₆) ; 1,50-1,61 (ma, 4H, H_{2'}) ; 3,40-3,56 (ma, 8H, H₁ et H_{1'}) ; 3,75 (dd, 2H, H₃ₐ) ; 4,08 (dd, 2H, H_{3b}) ; 4,28 (qt, 2H, H₂).
¹³C RMN (CDCl₃) : 25,47 (C₅) ; 26,10 (C_{2'}) ; 26,82 (C₆) ; 29,52 ; 29,60 ; 29,65 ; 29,66 ; 29,72 ; 29,73 (C_{3'} → C_{8'}) ; 66,97 (C₃) ; 71,85 ; 71,94 (C₁ et C_{1'}) ; 74,79 (C₂) ; 109,39 (C₄).

A l'issue de l'étape c), on obtient le 1,1-O-(hexadécane-1,16-diyl)-bis-(rac-glycérol) (rendement quantitatif) ayant les caractéristiques suivantes : 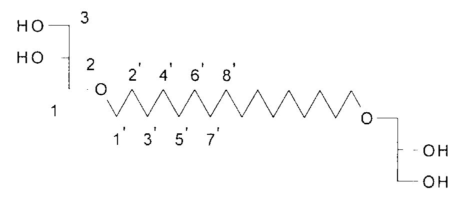
Solide blanc
M = 406,61 g.mol⁻¹ ; C₂₂H₄₆O₆
R_{F} = 0,77 (méthanol)
Point de fusion : 93-94°C (chloroforme)
¹H RMN (CD₃OD, 50°C) : 1,20-1,33 (ma, 24H, H_{3'} → H_{8'}) ; 1,45-1,52 (ma, 4H, H_{2'}) ; 3,32-3,40 (ma, 8H, H₁ et H_{1'}) ; 3,41 (dd, 2H, H₃ₐ) ; 3,48 (dd, 2H, H_{3b}) ; 3,67 (qt, 2H, H₂) ; 4,51 (I, 4H, OH).
¹³C RMN (CD₃OD, 50°C) : 28,05 (C_{2'}) ; 31,39 ; 31,50 ; 31,54 ; 31,59 (C_{3'} → C_{8'}) ; 65,69 (C₃) ; 73,21 (C₂) ; 73,57 (C₁) ; 74,24 (C_{1'}).
SM : 424,72 (100) [M + NH₄]⁺ ; 407,12 (44,4) [M + H]⁺

| | | | |
|---|---|---|---|
| Analyse élémentaire (C₂₂H₄₆O₆) | (% calculés) | C = 64,99 ; | H = 11,40 |
| | (% trouvés) | C = 64,82 ; | H = 11,66 |

### EXEMPLE 6

### a- Synthèse du méthanesulfonate de n-undéc-10-ynyle

Dans un ballon tricol de 25 ml contenant 1,68 g (10 mmoles) de 10-undécyn-1-ol (INTERCHIM) et 1,52 g (15 mmoles) de triéthylamine dans 10 ml de dichlorométhane, maintenu à une température de -10 à 0°C, on introduit goutte à goutte 1,26 g (11 mmoles) de chlorure de méthanesulfonyle.

Après une heure sous agitation, le mélange est lavé successivement par une solution d'HCl froid (10 % en poids), de Na₂CO₃ saturée, de NaCI saturée et enfin à l'eau distillée.

Après séchage (MgSO₄), élimination du solvant et distillation sous vide, on récupère le méthanesulfonate de n-undéc-10-ynyle (rendement : 90 %). Les caractéristiques de ce composé sont les suivantes : 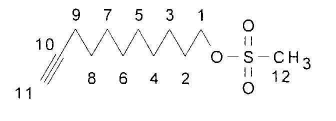
Liquide légèrement jaune.
M = 246,37 g.mol⁻¹ ; C₁₂H₂₂O₃S
R_{F} = 0,40 (éther de pétrole/éther; 1/1 v:v)
Point d'ébullition = 140-142°C/1 mm Hg
¹H RMN (CDCl₃) : 1,23-1,40 (ma, 12H, H₃ → H₈) ; 1,71-1,79 (ma, 2H, H₂) ; 1,94 (t, 1H, H₁₁, ⁴J_{H11-H9} = 2,54) ; 2,18 (dt, 2H, H₉, J_{H9-H8} = 6,6 ; ⁴J_{H9-H11} = 2,54) ; 3,00 (s, 3H, H₁₂) ; 4,22 (t, 2H, H₁, J_{H1-H2} = 6,6).
¹³C RMN (CDCl₃) : 18,38 (C₉) ; 25,40 (C₃₎ ; 28,42 ; 28,64 ; 28,96 ; 29,12 ; 29,26 (C₂ et C₄→ C₈) ; 37,36 (C₁₂) ; 68,17 (C₁₁) ; 70,22 (C₁) ; 84,72 (C₁₀).

### b- Synthèse du 1-O-(undéc-10-ynyl)-rac-isopropylidèneglycérol

On procède dans les conditions de l'exemple 1 (étape b) modifié en ce que l'on utilise le méthanesulfonate de n-undéc-10-ynyle.

On obtient le 1-O-(undéc-10-ynyl)-rac-isopropylidèneglycérol (rendement : 72 %) ayant les caractéristiques suivantes : 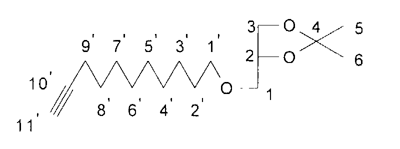
Liquide incolore
M = 282,43 g.mol⁻¹ ; C₁₇H₃₀O₃
R_{F} = 0,83 (éther de pétrole/éther ; 1/1 v:v)
Point d'ébullition : 138-140°C/2 mm Hg
¹H RMN (CDCl₃) : 1,25-1,40 (ma, 12H, H_{3'} → H_{8'}) ; 1,36 (s, 3H, H₅) ; 1,42 (s, 3H, H₆) ; 1,52-1,61 (ma, 2H, H_{2'}) ; 1,93 (t, 1H, H_{11'},⁴J_{H11'-H9'} =2,54) ; 2,18 (dt, 2H, H_{9'}, J_{H9'-H8'} = 6,6 ; ⁴J_{H9'-H11'} = 2,54) ; 3,33-3,51 (ma, 4H, H₁ et H_{1'}) ; 3,73 (dd, 1H, H₃ₐ, J_{H3a-H3b} = 8,2 ; J_{H3a-H2} = 6,4) ; 4,06 (dd, 1H, H_{3b}, J_{H3b-H3a} = 8,2 ; J_{H3b-H2} = 6,4) ; 4,27 (qt, 1H, H₂)
¹³C RMN (CDCl₃) : 18,38 (C_{9'}) ; 25,42 (C₅) ; 26,02 (C_{2'}) ; 26,77 (C₆) ; 28,46; 28,72; 29,03; 29,38; 29,41 ; 29,53 (C_{3'} → C_{8'}) ; 66,91 (C₃) ; 68,08 (C_{11'}) ; 71,81 et 71,85 (C₁ et C_{1'}) ; 74,74 (C₂) ; 84,75 (C_{10'}) ; 109,35 (C₄).

### c- Synthèse du 1,1-O-(docosa-10,12-diyne-1,22-diyl)-bis-(rac-isopropylidèneglycérol)

Dans un ballon tricol de 100 ml muni d'un réfrigérant, on introduit 1,56 g (7,8 mmoles) de diacétate de cuivre monohydraté et 36 ml de pyridine. Le mélange est chauffé à 55°C pendant une heure sous agitation, et on ajoute goutte à goutte 1,69 g (6 mmoles) de 1-O-(undéc-10-ynyl)-rac-isopropylidèneglycérol en solution dans 4 ml de pyridine.

Après deux heures, le mélange est refroidi et filtré (verre fritté).

Au résidu obtenu, de couleur verte, on ajoute 30 ml d'éther de pétrole et on le filtre à nouveau. Cette opération et répétée plusieurs fois. Les filtrats sont rassemblés, lavés successivement avec de l'eau, une solution d'acide chlorhydrique (2N) et de l'eau.

Après séchage (MgSO₄) et élimination du solvant par distillation, on récupère 1,65 g d'un mélange contenant le composé de départ et le 1,1-O-(docosa-10,12-diyne-1,22-diyl)-bis-(rac-isopropylidèneglycérol), ce dernier composé étant isolé par chromatographie sur une colonne de gel de silice (éluant : gradient éther de pétrole/éther) avec un rendement de 54 %. Les caractéristiques de ce composé sont les suivantes : 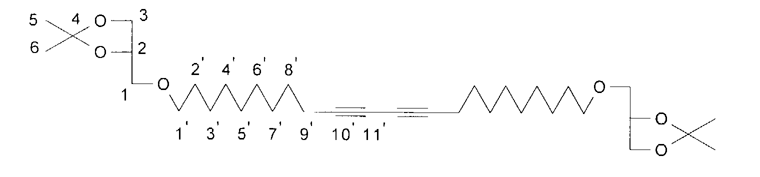
Huile incolore
M = 562,84 g.mol⁻¹ ; C₃₄H₅₈O₆
R_{F} = 0,62 (éther de pétrole/éther ; 1/1 v:v)
¹H RMN (CDCl₃) : 1,25-1,31 (ma, 20H, H_{3'} → H_{7'}) ; 1,36 (s, 6H, H₅) ;1,42 (s, 6H, H₆) ; 1,51 (qt, 4H, H_{8'}) ; 1,57 (qt, 4H, H_{2'}) ; 2,24 (t, 4H, H_{9'}) ; 3,40-3,53 (ma, 8H, H₁ et H_{1'}) ; 3,73 (dd, 2H, H₃ₐ) ; 4,06 (dd, 2H, H_{3b}) ; 4,27 (qt, 2H, H₂).
¹³C RMN (CDCl₃) : 19,20 (C_{9'}) ; 25,43 (C₅) ; 26,02 (C_{2'}) ; 26,78 (C₆) ; 28,33; 28,82 ; 29,03 ; 29,39; 29,54 (C_{3'} → C_{8'}) ; 65,24 (C_{11'}) ; 66,93 (C₃) ; 71,82; 71,86 (C₁ et C_{1'}) ; 74,75 (C₂) ; 77,53 (C_{10'}) ; 109,36 (C₄).

### d- Synthèse du 1,1-O-(docosane-1,22-diyl)-bis-(rac-glycérol)

Dans un ballon de 50 ml, on introduit 0,85 g (1,51 mmole) de 1,1-O-docosa-10,12-diyne-1,22-diyl)-bis-(rac-isopropylidèneglycérol), 64 mg de catalyseur palladium/charbon (10 % en poids de palladium) et 20 ml de méthanol.

Le mélange obtenu est agité sous atmosphère d'hydrogène à 20°C pendant 12 heures. Après filtration et élimination du méthanol, le résidu obtenu est solubilisé dans 30 ml d'éther éthylique et séché (MgSO₄). Après élimination du solvant, le 1,1-O-(docosane-1,22-diyl)-bis-(rac-isopropylidèneglycérol) obtenu (1,5 mmole) est hydrolysé en présence de 56 mg (0,3 méq.) de résine DOWEX 50X8 (ACROS ORGANICS) dans 3 ml d'acétonitrile. Le 1,1-O-(docosane-1,22-diyl)-bis-(rac-glycérol) obtenu (rendement : 91 %) présente les caractéristiques suivantes : 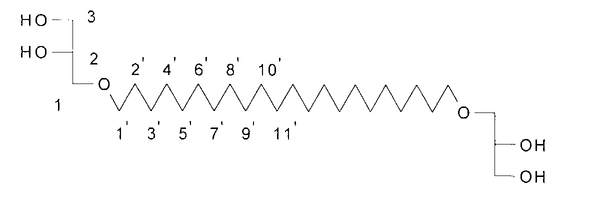
Solide blanc
M = 490,77 g.mol⁻¹ ; C₂₈H₅₈O₆
R_{F} = 0,78 (méthanol)
Point de fusion : 110°C (chloroforme)
¹H RMN (CD₃OD, 50°C) : 1,21-1,33 (ma, 36H, H_{3'} → H_{11'}) ; 1,45-1,52 (ma, 4H, H_{2'}) ; 3,34-3,43 (ma, 8H, H₁ et H_{1'}) ; 3,44 (dd, 2H, H₃ₐ) ; 3,52 (dd, 2H, H_{3b}) ; 3,68 (qt, 2H, H₂) ; 4,49 (I, 4H, OH).
¹³C RMN (CD₃OD, 50°C) : 28,12 (C_{2'}) ; 31,43 ; 31,55 ; 31,57 ; 31,58 ; 31,61 (C_{3'} → C_{11'}) ; 65,68 (C₃) ; 73,25 (C₂) ; 73,61 (C₁) ; 74,27 (C_{1'}).
SM : 508,25 (100) [M + NH₄]⁺ ; 491,28 (28,0) [M + H]⁺

| | | | |
|---|---|---|---|
| Analyse élémentaire (C₂₈H₅₈O₆) | (% calculés) | C = 68,53 ; | H = 11,91 |
| | (% trouvés) | C = 68,45 ; | H = 11,98 |

### EXEMPLE 7

### a- Synthèse du méthanesulfonate de n-undéc-10-ynyle

On procède dans les conditions de l'exemple 6 (étape a).

### b- Synthèse du glycérol-1,3-dibenzyl-2-(undéc-10-ynyl)triéther

On procède dans les conditions de l'exemple 2 (étape b) modifié en ce que l'on utilise le méthanesulfonate de n-undéc-10-ynyle et le glycérol-1,3-dibenzyléther.

On obtient le glycérol-1,3-dibenzyl-2-(undéc-10-ynyl) triéther (rendement : 43 %) ayant les caractéristiques suivantes : 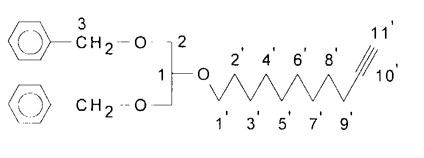 Liquide légèrement jaune
M = 422,61 g.mol⁻¹ ; C₂₈H₃₈O₃
R_{F} = 0,80 (éther de pétrole/éther ; 1/1 v:v)
¹H RMN (CDCl₃) : 1,25-1,39 (ma, 12H, H_{3'} → H_{8'}) ; 1,53-1,61 (ma, 2H, H_{2'}) ; 1,93 (t, 1H, H_{11'}, ⁴J_{H11'-H9'} = 2,54) ; 2,17 (dt, 2H, H_{9'}, J_{H9'-H8'} = 7,1, ⁴J_{H9}'_{H11'} = 2,54) ; 3,53-3,65 (ma, 7H, H₁, H₂ et H_{1'}) ; 4,54 (s, 4H, H₃) ; 7,24-7,33 (ma, 10H, H_{arom.}).
¹³C RMN (CDCl₃) : 18,38 (C_{9'}) ; 26,06 (C_{2'}) ; 28,47 ; 28,73 ; 29,03 ; 29,39 ; 29,42 ; 29,45 (C_{3'} → C_{8'}) ; 68,08 (C_{11'}) ; 70,14 (C₂) ; 70,58 (C_{1'}) ; 73,36 (C₃) ; 77,93 (C₁) ; 84,74 (C_{10'}) ; 127,53 ; 127,60 ; 128,11 ; 128,31 ; 138,36 (C_{arom.}).

### c- Synthèse du 2,2-O-(docosa-10,12-diyne-1,22-diyl)-bis-(1,3-dibenzylglycérol)

On procède dans les conditions de l'exemple 6 (étape c) modifié en ce que l'on utilise le glycérol-1,3-dibenzyl-2-(undéc-10-ynyl) triéther.

On obtient le 2,2-O-(docosa-10,12-diyne-1,22-diyl)-bis-(1,3-dibenzylglycérol) (rendement : 35 %) ayant les caractéristiques suivantes : 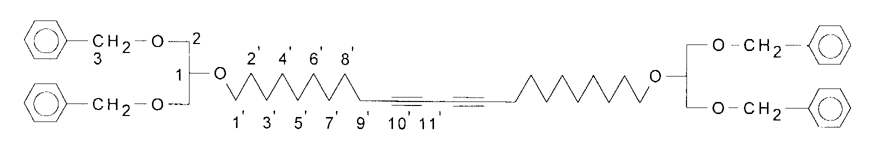
Huile incolore
M = 825,06 g.mol⁻¹ ; C₅₆H₅₆O₆
R_{F} = 0,66 (éther de pétrole/éther ; 1/1 v:v)
¹H RMN (CDCl₃) : 1,24-1,31 (ma, 20H, H_{3'} → H_{7'}) ; 1,50 (qt, 4H, H_{8'}) ;1,57 (qt, 4H, H_{2'}) ; 2,23 (t, 4H, H_{9'}) ; 3,37-3,55 (ma, 14H, H₁, H₂ et H_{1'}) ; 4,55 (s, 8H, H₃) ; 7,26-7,34 (ma, 20H, H_{arom.}).
¹³C RMN (CDCl₃) : 19,28 (C_{9'}) ; 26,15 (C_{2'}) ; 28,42 ; 28,92 ; 29,13 ; 29,51 ; 30,15 (C_{3'} → C_{8'}) ; 65,33 (C_{11'}) ; 70,21 (C₂) ; 70,65 (C_{1'}) ; 73,44 (C₃) ; 77,63 (C_{10'}) ; 78,00 (C₁) ; 127,61; 127,68 ; 128,40 ; 138,43 (C_{arom.}).

### d- Synthèse du 2,2-O-(docosane-1,22-diyl)-bis-(glycérol)

Dans un ballon de 50 ml, on introduit 0,3 mmole de 2,2-O-(docosane-10,12-diyne-1,22-diyl)-bis-(1,3-dibenzylglycérol), (7,5 % poids/poids) de catalyseur palladium/charbon (10 % en poids de palladium) et 5 ml de méthanol.

Le mélange obtenu est agité sous atmosphère d'hydrogène à 20°C pendant 12 heures. Après filtration à 50°C et élimination du méthanol, on récupère le 2,2-O-(docosane-1,22-diyl)-bis-(glycérol) (rendement : 91 %) ayant les caractéristiques suivantes : 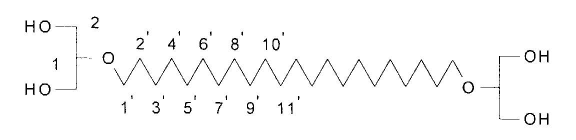
Solide blanc
M = 490,77 g.mol⁻¹ ; C₂₈H₅₈O₆
R_{F} = 0,78 (méthanol)
Point de fusion : 115°C (chloroforme)
¹H RMN (CD₃OD, 50°C) : 1,20-1,32 (ma, 36H, H_{3'} → H_{11'}) ; 1,45-1,52 (ma, 4H, H_{2'}) ; 3,43-3,55 (ma, 14H, H₁, H₂ et H_{1'}) ; 4,52 (I, 4H, OH).
¹³C RMN (CD₃OD, 50°C) : 28,10 (C_{2'}) ; 31,49 ; 31,56 ; 31,60 ; 31,63 ; 32,05 (C_{3'} → C_{11'}) ; 63,38 (C₂) ; 72,33 (C_{1'}) ; 83,25 (C₁).
SM : 508,32 (100) [M + NH₄]⁺ ; 491,25 (39,1) [M + H]⁺

| | | | |
|---|---|---|---|
| Analyse élémentaire (C₂₈H₅₈O₆) | (% calculés) | C = 68,53 ; | H = 11,91 |
| | (% trouvés) | C = 68,38 ; | H = 11,90 |

## Revendications

1. Composés bolaformes de formule :
R - O - A - O - R
dans laquelle :
R, identiques, représentent un motif ou un enchaînement de motifs de formule : 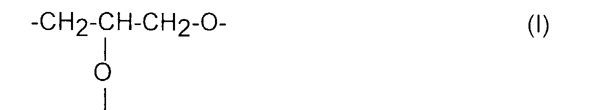 et/ou 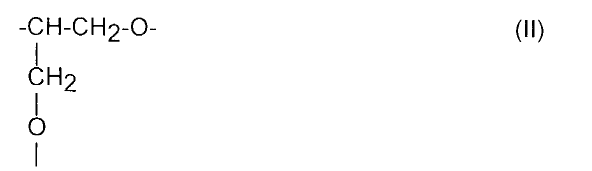 formules dans lesquelles la valence disponible de l'atome d'oxygène est liée à un atome d'hydrogène,
A représente une chaîne hydrocarbonée linéaire ou ramifié, saturée ou insaturée, renfermant 6 à 32 atomes de carbone,
à l'exception des composés dans lesquels R représentent -CH₂-CH(OH)-CH₂OH et A est une chaîne hydrocarbonée linéaire saturée renfermant 16, 20 ou 32 atomes de carbone.

2. Composés selon la revendication 1, caractérisés en ce que A renferme 14 à 24 atomes de carbone.

3. Procédé de préparation de composés de formule R-O-A-O-R dans laquelle R, identiques, représentent un motif ou un enchaînement de motifs de formule : 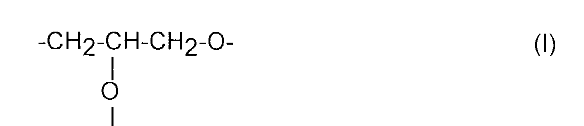 et/ou 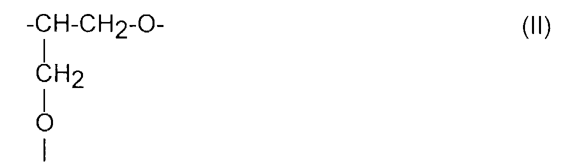 formules dans lesquelles la valence disponible de l'atome d'oxygène est liée à un atome d'hydrogène,
A représente une chaîne hydrocarbonée linéaire ou ramifié, saturée ou insaturée, renfermant 6 à 32 atomes de carbone,
ledit procédé étant caractérisé en ce qu'il comprend les étapes suivantes :
***a-*** réaction d'un α,ω-alcanediol, linéaire ou ramifié, saturé ou insaturé en C₆-C₃₂ et d'un composé de formule R'SO₂Cl dans laquelle R' représente un radical alkyle, linéaire ou ramifié, aryle, alkylaryle ou arylalkyle,
***b-*** réaction du produit de l'étape a- et d'un composé de formule R-OH dans laquelle R représente un motif ou un enchaînement de motifs de formule (I) et/ou (II) tels que définis précédemment, la valence disponible de l'atome d'oxygène étant liée à un groupement protecteur, et
***c-*** déprotection

4. Procédé selon la revendication 3, caractérisé en ce que l'on utilise le chlorure de méthanesulfonyle ou le chlorure de p-toluènesulfonyle.

5. Procédé selon l'une des revendications 3 ou 4, caractérisé en ce que le composé R-OH est l'isopropylidèneglycérol, le diisopropylidènetriglycérol ou le glycérol-1,3-dibenzyléther.

6. Procédé selon l'une des revendications 3 à 5, caractérisé en ce que le α, ω-alcanediol renferme 14 à 24 atomes de carbone.

7. Procédé de préparation de composés de formule R-O-A-O-R dans laquelle :
R, identiques, représentent un motif ou un enchaînement de motifs de formule : 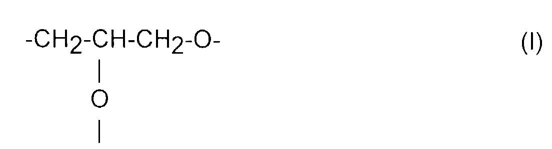 et/ou 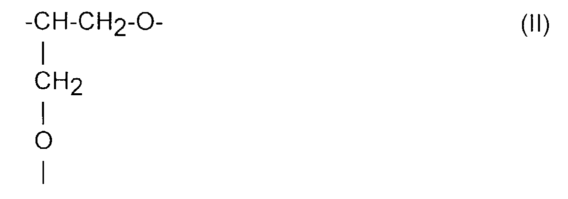 formules dans lesquelles la valence disponible de l'atome d'oxygène est liée à un atome d'hydrogène,
A représente une chaîne hydrocarbonée linéaire ou ramifié, saturée ou insaturée, renfermant 6 à 32 atomes de carbone,
ledit procédé étant caractérisé en ce qu'il comprend les étapes suivantes :
***a-*** réaction d'un alcool ω-acétylénique en C₄-C₁₆ et d'un composé de formule R'SO₂Cl, R' ayant la signification donnée ci-avant,
***b-*** réaction du produit de l'étape a- et d'un composé de formule R-OH ayant la signification donnée ci-avant,
***c-*** couplage oxydant en présence d'un sel de cuivre,
***d-*** réduction, et, le cas échéant, déprotection.

8. Procédé selon la revendication 7, caractérisé en ce que l'on utilise le chlorure de méthanesulfonyle ou le chlorure de p-toluènesulfonyle.

9. Procédé selon l'une des revendications 7 ou 8, caractérisé en ce que le composé R-OH est l'isopropylidèneglycérol, le diisopropylidènetriglycérol ou le glycérol-1,3-dibenzyléther.

10. Procédé selon l'une des revendications 7 à 9, caractérisé en ce que le sel de cuivre est choisi parmi les composés de formule CuX ou CuX₂ dans laquelle X représente Cl, Br, I, CH₃COO ou CN.

11. Procédé selon l'une des revendications 7 à 10, caractérisé en ce que l'alcool ω-acétylénique renferme 8 à 14 atomes de carbone.

12. Utilisation des composés selon l'une des revendications 1 ou 2 en tant qu'agent tensio-actif.

13. Utilisation des composés selon l'une des revendications 1 ou 2 en tant qu'agent d'encapsulation.

14. Utilisation des composés selon l'une des revendications 1 ou 2 en tant qu'agent d'extraction de protéines.
